# EUROPEAN PATENT SPECIFICATION

(11) **EP 3 852 781 B1**
(45) Date of publication and mention of the grant of the patent: **19.03.2025**
(21) Application number: 19782660.5
(22) Date of filing: 19.08.2019
(51) Int. Cl.: A61K 38/07

(54) **COMPOSITIONS COMPRISING ACYLATED TETRAPEPTIDES FOR DERMATOLOGICAL AND COSMETIC APPLICATIONS**
ACYLIERTE TETRAPEPTIDE ENTHALTENDE ZUSAMMENSETZUNGEN FÜR DERMATOLOGISCHE UND KOSMETISCHE ANWENDUNGEN
COMPOSITIONS COMPRENANT DES TETRAPEPTIDES ACYLÉS POUR DES APPLICATIONS DERMATOLOGIQUES ET COSMETIQUES

(30) Priority: 17.08.2018 GB 201813425
(43) Date of publication of application: 28.07.2021
(73) Proprietor: Miller, Norman E., City London EC1Y 2AN (GB); Miller, Irina, City London EC1Y 2AN (GB)
(72) Inventor: Miller, Norman E., City London EC1Y 2AN (GB); Miller, Irina, City London EC1Y 2AN (GB)
(74) Representative: Finnegan Europe LLP
(86) International application number: PCT/GB2019/000121
(87) International publication number: WO 2020/035651

(56) References cited:
- EP-A1- 2 065 029
- US-A1- 2004 132 667
- US-A1- 2006 275 237
- B. J. VAN LENTEN ET AL: "Anti-inflammatory apoA-I-mimetic peptides bind oxidized lipids with much higher affinity than human apoA-I", THE JOURNAL OF LIPID RESEARCH, vol. 49, no. 11, 1 January 2008 (2008-01-01), pages 2302 - 2311, XP055044809, ISSN: 0022-2275, DOI: 10.1194/jlr.M800075-JLR200
- MOHAMAD NAVAB ET AL: "Oral small peptides render HDL antiinflammatory in mice and monkeys and reduce atherosclerosis in ApoE null mice", CIRCULATION RESEARCH, GRUNE AND STRATTON, BALTIMORE, US, vol. 97, no. 6, 1 September 2005 (2005-09-01), pages 524 - 532, XP002691337, ISSN: 0009-7330, [retrieved on 20050811], DOI: 10.1161/01.RES.0000181229.69508.2F
- JIE CHEN ET AL: "Computer-aided design of short peptide ligands targeting tumor necrosis factor-alpha for adsorbent applications", JOURNAL OF MATERIALS CHEMISTRY B, vol. 6, no. 26, 7 June 2018 (2018-06-07), GB, pages 4368 - 4379, XP055667901, ISSN: 2050-750X, DOI: 10.1039/C8TB00563J
- "High Density Lipoproteins. Handbook of Experimental Pharmacology, vol 224. Springer, Cham First Online 01 December 2014 DOI https://doi.org/10.1007/978-3-319-09665-0_21OLITCiteNPL Publisher Name Springer, Cham", 1 December 2014, SPRINGER, ISBN: 978-3-319-09665-0, article R M STOEKENBROEK ET AL: "ApoA-I mimetics", pages: 631 - 648, XP055667977, DOI: https://doi.org/10.1007/978-3-319-09665-0

## Description

The present invention relates to an improved skin care composition comprising an acylated peptide of formula (I), an acylated peptide, and a method of protecting skin from damage.

It has been known for many years that lipid peroxides (also known as lipid hydroperoxides or lipoperoxides) are produced in skin when it is exposed to sunlight. This is due not only to the UVB wavelength as once thought, but also to the UVA, infra-red, and visible wavelengths. Recent research has discovered that several atmospheric pollutants (ozone, nitrogen dioxide, particulate matter, and polycyclic hydrocarbons) and cigarette smoke also generate lipid peroxides in skin.

Lipid peroxides are considered to be an important cause of the changes that occur in skin during aging. This is true of both the cosmetic effects (such as wrinkles, roughness, thinning, and brown blotches) and all three major types of skin cancer, which are increasing in incidence worldwide and are collectively the most common form of cancer.

Lipid peroxides are produced by the oxidation of lipids by reactive oxygen species (ROS). It is by generating ROS in skin that solar radiation (such as sunlight), atmospheric pollutants, and cigarette smoke induce the formation of lipid peroxides. Lipid peroxides and certain molecules derived from them (e.g. MDA, 4-HNE) are toxic to cell membranes, damage structural proteins between cells (e.g. collagen and elastin), inhibit certain anti-oxidant enzymes (e.g. superoxide dismutase), increase the secretion by cells of matrix metalloproteinases (which digest collagen), and damage DNA. All these effects are thought to contribute to the cosmetic changes in skin during aging, while damage to DNA is the cause of all types of cancer. Lipid peroxides also contribute to the redness, soreness, and pain in skin (sunburn) after severe exposure to sunlight. Lipid peroxides have acute effects (e.g. inflammation, pain and sunburn) and chronic effects (e.g. damage to cells, proteins, DNA; altered enzyme activities; aging of skin; and cancer).

Sun protection creams and lotions attempt to reduce the damaging effects of sunlight by reflecting it, absorbing it, or inhibiting oxidative processes (e.g. alpha-tocopherol). However, even in combination they are only partially effective, and then usually for only a few hours. Some skin care products attempt to protect against atmospheric pollution by creating a barrier on the surface, but there is little evidence that this is effective.

There are no skin care products that attempt to prevent the harmful effects of lipid peroxides once formed in skin. US2004132667 and EP2065029 disclose acylated tetrapeptides and tripeptides for cosmetic skin care.

A way of ameliorating these problems has been sought.

According to the invention there is provided a composition comprising (a) an acylated peptide of formula (I)

R-X¹-X²-X³-X⁴ (I)

wherein R represents a fatty acid residue having a chain length suitable for assisting the compound of formula (I) to penetrate skin, X¹ represents a basic amino acid or a hydrophobic aromatic amino acid, X² represents a basic amino acid, X³ represents an acidic amino acid, and X⁴ represents a hydrophobic aliphatic amino acid or polar neutral amino acid, a dermatologically acceptable salt, solvate, and/or enantiomer thereof; and (b) a dermatologically acceptable carrier.

According to the invention there is also provided an acylated peptide of formula (II)

R¹-X¹-X²-X³-X⁴ (II)

wherein R¹ represents a fatty acid residue having a chain length suitable for assisting the compound of formula (II) to penetrate skin, and wherein X¹, X², X³, and X⁴ are as defined above with respect to the peptide of formula (I), a dermatologically acceptable salt, solvate, and/or enantiomer thereof, which is an acylated peptide of formula (IIA) or (IIB)

KRES (IIA)

FREL (IIB)

wherein the acyl substituent group is a fatty acid residue having a chain length suitable for assisting the acylated peptide of formula (IIA) or (IIB) to penetrate skin.

According to the invention, there is provided an acylated peptide of formula (I) as defined above for use in inhibiting or preventing the development of a skin condition.

According to the invention there is further provided a method of inhibiting or preventing the development of a skin condition which method comprises the step of applying a peptide of formula (I) to a human or animal in need of such protection.

The advantages of the invention include that the damaging effects that lipid peroxides can cause in skin may be partially or completely prevented using the peptide, composition, and/or method according to the invention by using the peptide to sequester the lipid peroxides; the peptide is small enough to be able to penetrate the epidermis and dermis; and fatty acylation of the peptide will assist with penetration into the skin by rendering the peptide more lipophilic. Further advantages include improved membrane integrity and function of cells in the epidermis and dermis; increased activity of anti-oxidant enzymes; diminished secretion of matrix metalloproteinases by fibroblasts (reducing breakdown of collagen); reduced protein damage by MDA and 4-HNE and other reactive aldehydes derived from lipid peroxides; and reduced DNA damage by MDA and 4-HNE and other reactive aldehydes. Penetration of the skin will reduce washing off with sweating and swimming. The peptides will produce no reduction of tanning or of vitamin D production (skin being an important source of the vitamin). Additionally, protection will be against all wavelengths of sunlight known to damage skin.

Several peptides are in use in skin care products for their cosmetic effects, although for most of them few or no efficacy data are available from clinical studies. One with proven efficacy is palmitoyl pentapeptide-4 (Matrixyl^{™}), Pal-Lys-Thr-Thr-Lys-Ser (pal-KTTKS), which stimulates the production in skin of collagen, elastin, fibronectin, and glycosaminoglycan. Its effectiveness in reducing skin wrinkles has been demonstrated in double blind placebo- controlled clinical studies. Another is palmitoyl tripeptide-3/5, Pal-Lys-Val-Lys (pal-KVK), which mimics the effects of the extracellular matrix protein, thrombospondin-1, resulting in increased collagen synthesis, and has been shown to reduce wrinkles and increase skin smoothness. Tetrapeptide-21, Gly-Glu-Lys-Gly (GEKG), increases collagen and other matrix proteins and was shown to improve skin elasticity to a similar degree to that achieved with palmitoyl pentapeptide-4. Acetyl hexapeptide-3, Acetyl-Glu-Glu-Met-Gln-Arg-Arg (acetyl-EEMQRR), Argireline^{™}, inhibits the release of neurotransmitters in skin to produce a transient 'Botox-like' effect (relaxation of subcutaneous muscle). None of the peptides mentioned above or any other peptides used in skin care products is known to sequester lipid peroxides.

The potential contribution of the pro-inflammatory properties of lipid peroxides to the pathogenesis of diseases, in particular those of the cardiovascular system, has been discussed (Navab M. Circ Res 2005; 97: 524-32). However, no consideration has been given to their role in the age-related changes in skin and their cosmetic effects, which are not consequences of inflammation but of changes in DNA integrity, collagen breakdown, elastin synthesis, and enzyme activities. The ageing of tissues, including those of skin, is not a disease but an inevitable transformation (Rattan SIS. Aging and Disease 2014; 5: 196-202), albeit one whose rate can be influenced by environmental factors.

In principle, the accumulation of lipid peroxides to prevent their long-term effects on the composition and structure of skin could be prevented by one or more of three mechanisms: lowering their production, increasing their breakdown, or sequestering them in a way that eliminates their biological activity. Production can be partially reduced by sunscreens and antioxidants, such as vitamin E (Rinnerthaler M. Biomolecules 2015; 5, 545-589). Theoretically, breakdown might be enhanced by application of appropriate enzymes, though several would be needed given the variable nature of lipid peroxides. Furthermore, they are unlikely to enter skin when applied topically, and the effects of the resultant end-products are unknown. Molecular sequestration, on the other hand, has several attractions.

The essential properties of an effective sequestrant of lipid peroxides in skin are that it both (a) has a high binding affinity for them and (b) can enter the epidermis and superficial dermis. The two principal determinants of penetration into skin are molecular size and lipophilicity. The smaller the size and the greater the lipophilicity of a molecule, the greater the penetration. An acylated tetrapeptide that irreversibly binds lipid peroxides is ideally suited to this purpose. Furthermore, such a molecule will have the additional property of binding to the surface membranes of cells (Resh MD. Current Biology 2013; 23: R431-5), thereby preventing its entry into the blood and ensuring that the peptide-peroxide complex is eventually shed during the normal desquamation of keratinocytes. This mechanism also avoids the consequential production of metabolites whose acute and chronic effects are unknown. Ideally, such an agent should also be biodegradable, be unlikely to provoke allergy during long-term use, and cause no reduction of vitamin D production in skin.

The sequential processes of entry into the skin, sequestration of lipid peroxides, binding to cell surfaces, and eventual loss by desquamation provide a mechanism whereby such a molecule physically "cleanses" the skin without deleterious local or systemic biochemical effects.

Apolipoprotein mimetic peptides (AMPs) are short chains of amino acids that mimic some of the properties of apolipoproteins, the protein components of plasma lipoproteins (the particles that carry fats in blood). They first attracted the interest of cardiovascular disease researchers on account of their ability to bind cholesterol and lipid peroxides, focal accumulations of which in artery walls contribute to the development of atherosclerosis (the cause of coronary artery disease). When cholesterol and lipid peroxides are sequestered by AMPs, they are rendered harmless.

The binding affinities of AMPs for lipid peroxides in vitro are many thousands of times greater than those of apolipoproteins (Van Lenten et al, J Lipid Res 2008; 49: 2302). The binding of non-oxidized lipids by AMPs does not interfere with the binding of lipid peroxides. AMPs do not occur naturally, and none has been developed commercially.

Most academic research on AMPs has been done with peptides that mimic the activities of a particular apolipoprotein called apo AI, the principal protein component of high-density lipoproteins (the carriers of so-called 'good' cholesterol). As apo Al seems to remove cholesterol from artery walls and take it to the liver, it was hoped that intravenous infusion of an AMP mimicking its effects would reverse atherosclerosis. However, no AMP has been successfully developed for this or any other use.

The AMP that originally attracted most interest among cardiovascular researchers was an 18 amino acid chain with the following amino acid sequence, which was named 18A: DWLKAFYDKVAEKLKEAF (Anantharamaiah et al, J Biol Chem 1985; 260: 10248).

Subsequent work by academic groups produced progressively smaller AMPs of just a few amino acids with similar biological activities, including two peptides having four amino acids: Lys-Arg-Glu-Ser (KRES) and Phe-Arg-Glu-Leu (FREL) (Navab et al, Circ Res 2005; 97: 524).

At a time when the pharmaceutical industry was interested in the potential of AMPs for reversing coronary artery disease, an 18 amino acid AMP closely resembling the 18A peptide was infused intravenously into humans in Phase 1 clinical studies, but no further studies were carried out. Interest dropped when it was found it was rapidly cleared from the blood (probably by the kidney, the physiological route of elimination of most natural peptides). No adverse effects were reported.

Although AMPs share some of the physico-chemical and biological properties of apolipoproteins, they do not share any parts of their amino acid sequences (i.e., they are not the equivalent of small fragments of the apolipoproteins). They are artificial molecules with no structural similarity to the proteins whose properties they share.

In some embodiments, R or R¹ may represent a fatty acid residue of formula RC(O)- where R represents a saturated or unsaturated straight or branched chain alkyl group having from 12 to 24 carbon atoms. In some embodiments, R may represent palmitoyl or myristoyl.

In some embodiments, X¹ may represent phenylalanine, tyrosine, tryptophan, histidine, lysine or arginine.

In some embodiments, X² may represent histidine, lysine or arginine.

In some embodiments, X³ may represent aspartic acid, or glutamic acid.

In some embodiments, X⁴ may represent alanine, valine, methionine, leucine, isoleucine, threonine, glutamine, cysteine, asparagine, or serine.

In some embodiments, the amino acid at position X⁴ may depend upon the type of amino acid that is present at position X¹. In some embodiments, where X¹ has a hydrophobic aromatic side chain (e.g. phenylalanine (F), tryptophan (W), or tyrosine (Y)), X⁴ may have a hydrophobic aliphatic side chain (e.g. leucine (L), alanine (A), isoleucine (I), methionine (M) or valine (V)). In some embodiments, where X¹ is a basic amino acid (e.g. lysine (K), histidine (H), or arginine (R)), X⁴ may have a polar neutral side chain (e.g. asparagine (N), cysteine (C), glutamine (Q), threonine (T), or serine (S)).

In some embodiments, the acylated peptide of formula (I) may be an acylated peptide of formula (IA) or (IB)

KRES (IA)

FREL (IB)

wherein one or more of the amino acids is subject to a conservative substitution and wherein the acyl substituent group is a fatty acid residue having a chain length suitable for assisting the peptide of formula (IA) or (IB) to enter the epidermis and dermis of skin. The acylated peptide of formula (II) is an acylated peptide of formula (IIA) or (IIB)

KRES (IIA)

FREL (IIB)

wherein one or more of the amino acids is subject to a conservative substitution and wherein the acyl substituent group is a fatty acid residue having a chain length suitable for assisting the acylated peptide of formula (IIA) or (IIB) to penetrate skin. A skilled person would be aware of what would constitute a conservative substitution of an amino acid in an acylated peptide of formula (IA) or (IB) or an acylated peptide of formula (IIA) or (IIB).

In some embodiments, the peptide of formula (I) may be of formula

Palmitoyl-KRES (SEQ ID NO: 3) (ID)

Myristoyl-KRES (SEQ ID NO: 5) (IID)

Palmitoyl-FREL (SEQ ID NO: 4) (IF)

Myristoyl-FREL (SEQ ID NO: 6) (IG)

In some embodiments, the peptide of formula (II) may be of formula

Palmitoyl-KRES (SEQ ID NO: 3) (IIC)

Myristoyl-KRES (SEQ ID NO: 5) (IID)

Palmitoyl-FREL (SEQ ID NO: 4) (IIE)

Myristoyl-FREL (SEQ ID NO: 6) (IIF)

In some embodiments, the method for inhibiting or preventing the development of a skin condition comprises the step of topically applying to a treatment surface of the body in need of such treatment a safe and effective amount of a peptide of formula (I) disclosed herein. The peptides, as defined above, include their dermatologically acceptable salts, solvates, and enantiomers.

The peptide and composition according to the invention are effective in preventing a wide variety of skin conditions in which lipid peroxides play a role, including but not limited to, sunburn, UV sensitivity, and the cosmetic effects associated with aging.

In some embodiments, a safe and effective amount of the peptides described herein is delivered topically to the skin of a human or animal (particularly a mammal). When administered topically, the peptides disclosed herein being small in molecular size and lipophilic are able to effectively penetrate the upper layers of the skin.

In addition, it will be appreciated that the benefits of the invention can be realized by combining application with one or more additional skin care agents. In some embodiments, the composition according to the invention may include an additional skin care agent. In some embodiments, the method according to the invention may additionally comprise application by an additional skin care agent. In some embodiments, the additional skin care agent may be one or more of a desquamatory agent, anti-acne agent, wrinkle repair agent, anti-oxidant, radical scavenger, chelator, anti-inflammatory agent, topical anaesthetic, anti-cellulite agent, flavonoid, antimicrobial agent, antifungal agent, sunscreen agent, and/or conditioning agent.

In some embodiments, the additional skin care agent comprises a sunscreen agent. Herein, "sunscreen agent" refers to an agent applied to the skin to prevent the acute and/or chronic deleterious effects of solar radiation either physically (reflection) or chemically (absorbance). Many sunscreens are known in the art and include one or more of titanium dioxide, zinc oxide, a PABA ester (such as glyceryl, padimate A and/or padimate O), a salicylate (such as homosalate or octyl salicylate), cinnamate (such as cinoxate, octyl methoxycinnamate, or octocrylene), benzophenone, and/or ecamsule.

In some embodiments, the method according to the invention comprises administering the peptide of formula (I) and the further skin care agent at substantially the same time. In some embodiments, the treatment with the peptide of formula (I) disclosed herein can precede or follow application of the further skin care agent by an interval ranging from minutes to weeks.

In some embodiments, the composition according to the invention may be a skin care composition comprising (a) a safe and effective amount of a peptide of formula (I), and (b) one or more dermatologically acceptable carriers.

Herein "dermatologically acceptable carrier" refers to a carrier which is suitable for topical application to the skin, has good aesthetic properties, is compatible with the actives disclosed herein and any other components, and will not cause any safety or toxicity concerns. In some embodiments a safe and effective amount of carrier is from about 50%, specifically from about 60%, more specifically from about 70% to about 99.99%, specifically to about 99.9%, and more specifically to about 98% by weight of the composition.

The dermatologically acceptable carrier used in the invention may be one of a wide variety of types. For example, suitable types include emulsion carriers, including, but not limited to, an oil-in-water, water-in-oil, water-in-oil-in-water, or oil-in-water-in-oil emulsion. In some embodiments, silicone can also be used as the oil, such that the potential emulsion carriers include silicone-in-water, water-in-silicone, water-in-silicone-in-water and oil-in-water-in-silicone emulsions. The dermatologically acceptable carrier may also take one of many forms, non-limiting examples of which include a liquid, milk, serum, lotion, cream, spray, aerosol, mousse, foam, stick, gel, and pencil. It may also contain liposomes or nanoparticles.

In some embodiments, an emulsion for use as the dermatologically acceptable carrier may include an aqueous solution and a lipid and/or oil. A suitable lipid and/or oil may be derived from an animal, plant, or petroleum and may be natural or synthetic (i.e., man-made, such as a silicone).

In some embodiments, an emulsion may also contain a humectant, such as glycerin. In some embodiments, an emulsion may also contain an emulsifier, for example an emulsifier in an amount from about 0.05%, more specifically from about 0.1% to about 10%, more specifically to about 5%, based on the weight of the composition. A suitable emulsifier may be non-ionic, anionic or cationic. Suitable emulsifiers are disclosed in, for example, McCutcheon's 2013 Emulsifiers and Detergents, North American Edition (2013). Suitable emulsions may have a wide range of viscosities, depending on the desired product form.

In some embodiments, the peptides of this patent, alone or in combination with each other, may be used at a concentration from about 0.1 to about 1000 ppm, preferably from 1 to 100 ppm in the composition or method according to the invention.

In some embodiments, the composition according to the invention may be a topical composition comprising as a dermatologically acceptable carrier from about 20% to about 50% by weight of the carrier of propylene glycol and from about 50% to about 80% by weight of the carrier of water, with pH adjusted to 4.0 with 10 mM citric acid.

In some embodiments, a skin condition to be prevented by the invention includes a cosmetic skin condition such as wrinkles, roughness, lentigenes (brown blotches), and sunburn.

The peptides of formula (I) and (II) disclosed herein may be synthesized from their constituent amino acids or their derivatives by conventional chemical synthesis in solution or solid-phase procedures based on the Fmoc/tBu approach and now widely used in the cosmetics and skin care industries (Amblard et al, Mol Biotechnol 2006; 33: 239), and purified by reverse phase HPLC. Alternatively, enzymatic synthesis can be used. Fatty acid acylation of peptides enhances penetration into the outer layers of skin by rendering them more lipophilic. Covalent attachment of a fatty acid (e.g. palmitic acid or myristic acid) to the N-terminal amino acid of small peptides is readily achieved by established chemical procedures (Lumbierres et al, Chemistry - A European Journal. 2005; 11; 7405).

The efficacy of a peptide will be assessed first in vitro and then in vivo in healthy humans. The invention will now be illustrated with reference to the following Examples which are not intended to limit the scope of the claimed invention.

### EXAMPLE 1

The testing of the efficacy of the peptide of formula (I) according to the invention *in vitro* will utilise monolayer cultures of normal human dermal keratinocytes and fibroblasts. Validated procedures for quantifying effects on metabolic changes induced by UVA (320-400 nm), UVB (280-320 nm), and infra-red A (near IR) (700-1400 nm) radiations will be employed. Measurements will include two or more of ROS production; messenger RNA abundances for matrix metalloproteinase-1, collagen and elastin; secretion of matrix metalloproteinase-1 into the culture medium; secretion of interleukin-1 and interleukin-6 (pro-inflammatory cytokines) into the medium; and established indicators of DNA damage. Additionally, one or more of the effects on cell viability, interleukin-1 production, and DNA damage (pyrimidine dimer production) may be measured using a commercial reconstructed full thickness human skin model (T-Skin^{™}) (Episkin, Lyon, France) which has been independently validated (Alépée et al (Toxicol in vitro 2015; 29: 1779).

### EXAMPLE 2

Clinical effects of the peptides will be assessed by four randomised double-blind placebo-controlled trials in groups of healthy women aged 30-65 years with each acting as her own control. In the first, the test peptide in a suitable carrier will be applied to the skin creases at the outer corner of one eye (so-called crow's foot) and a placebo consisting of the same amount of carrier without peptide will applied to the corresponding area of skin on the other side. Applications will be made morning and evening for 42 days, and measurements of wrinkle depth made at the start and end of this time by video-computer analysis of silicone 'fingerprints' of the skin surface, as described by Corcuff et al (Int. J. Cosm. Sci. 1985; 17: 126). The study will be carried out in 40-50 healthy subjects.

The effects on skin in vivo will be assessed also by a second double-blind trial in a different group of 40-50 healthy women in which the test peptide and placebo are applied to different sides of the face each morning and evening for 42 days. At the beginning and end of the treatment period, skin roughness will be measured by fast optical *in vivo* topometry (3-D imaging) as described by Kaczvinsky JR et al (J Cosmet Dermatol 2009; 8: 228).

In a third trial, the test preparation and placebo will be applied to the left and right forearms of 10 healthy women aged 30-65 years twice daily for 42 days. Biopsies from corresponding areas of skin on each side will be taken at the beginning and end of the study and assayed for pyrimidine dimers and 8-oxo-7, 8-dihydro-2-deoxyguanosine (indicators of DNA damage) as described by Emanuele et al (J Drugs Dermatol 2014; 13: 309).

## Claims

1. A composition comprising (a) an acylated peptide of formula (I)
R-X¹-X²-X³-X⁴ (I)
wherein R represents a fatty acid residue having a chain length suitable for assisting the compound of formula (I) to penetrate skin, X¹ represents a basic amino acid or a hydrophobic aromatic amino acid, X² represents a basic amino acid, X³ represents an acidic amino acid, and X⁴ represents a hydrophobic aliphatic amino acid or polar neutral amino acid, a dermatologically acceptable salt, solvate, and/or enantiomer thereof; and (b) a dermatologically acceptable carrier.

2. A composition as defined in Claim 1 wherein X¹ represents phenylalanine, tyrosine, tryptophan, histidine, lysine or arginine; X² represents histidine, lysine or arginine; X³ represents aspartic acid, or glutamic acid; and/or X⁴ represents alanine, valine, methionine, leucine, isoleucine, threonine, glutamine, cysteine, asparagine, or serine.

3. A composition as defined in Claim 1 or Claim 2 wherein X¹ has a hydrophobic aromatic side chain, X⁴ has a hydrophobic aliphatic side chain, or wherein X¹ is a basic amino acid, X⁴ has a polar neutral side chain.

4. A composition as defined in any one of the preceding Claims wherein R represents a fatty acid residue of formula R²C(O)- where R² represents a saturated or unsaturated straight or branched chain alkyl group having from 12 to 24 carbon atoms; preferably R represents palmitoyl or myristoyl.

5. A composition as defined in any one of the preceding Claims wherein the acylated peptide of formula (I) may be an acylated peptide of formula (IA) or (IB)
KRES (IA)
FREL (IB)
wherein the optional acyl substituent group is a fatty acid residue having a chain length suitable for assisting the peptide of formula (IA) or (IB) to enter the epidermis and dermis of skin.

6. A composition as defined in any one of the preceding Claims wherein the peptide of formula (I) may be of formula
Palmitoyl-KRES (SEQ ID NO: 3) (ID)
Myristoyl-KRES (SEQ ID NO: 5) (IID)
Palmitoyl-FREL (SEQ ID NO: 4) (IF)
Myristoyl-FREL (SEQ ID NO: 6) (IG)

7. A composition as defined in any one of the preceding Claims which includes an additional skin care agent; preferably the additional skin care agent is one or more of a desquamatory agent, anti-acne agent, wrinkle repair agent, anti-oxidant, radical scavenger, chelator, anti-inflammatory agent, topical anaesthetic, anti-cellulite agent, flavonoid, antimicrobial agent, antifungal agent, sunscreen agent, and/or conditioning agent, preferably wherein the additional skin care agent comprises a sunscreen agent.

8. A composition as defined in any one of the preceding Claims which is a skin care composition comprising (a) a safe and effective amount of a peptide of formula (I), and (b) one or more dermatologically acceptable carriers.

9. A composition as defined in any one of the preceding Claims which comprises a peptide of formula (I) at a concentration from about 0.1 ppm to about 1000 ppm.

10. An acylated peptide of formula (II)
R¹-X¹-X²-X³-X⁴ (II)
wherein R¹ represents a fatty acid residue having a chain length suitable for assisting the compound of formula (II) to penetrate skin, and wherein X¹, X², X³, and X⁴ are as defined in any one of claims 1 to 4 with respect to the peptide of formula {I), a dermatologically acceptable salt, solvate, and/or enantiomer thereof, a dermatologically acceptable salt, solvate, and/or enantiomer thereof, which is an acylated peptide of formula (IIA) or (IIB)
KRES (IIA)
FREL (IIB)
wherein the acyl substituent group is a fatty acid residue having a chain length suitable for assisting the acylated peptide of formula (IIA) or (IIB) to penetrate skin.

11. An acylated peptide as defined in Claim 10 which is of formula
Palmitoyl-KRES (SEQ ID NO: 3) (IIC)
Myristoyl-KRES (SEQ ID NO: 5) (IID)
Palmitoyl-FREL (SEQ ID NO: 4) (IIE)
Myristoyl-FREL (SEQ ID NO: 6) (IIF)

12. An acylated peptide as defined in Claim 10 wherein R¹ represents a fatty acid residue of formula R₂C(O)- where R₂ represents a saturated or unsaturated straight or branched chain alkyl group having from 12 to 24 carbon atoms.

13. An acylated peptide of formula (I) as defined in any one of Claims 1 to 6 for use in inhibiting or preventing the development of a skin condition.

14. The composition of claims 1-6 for use in inhibiting or preventing the development of a skin condition, whereby the composition as defined in any of claims 1 to 6 is applied to a human or animal in need of such protection, whereby optionally a safe and effective amount of the composition as defined in any one of claims 1 to 7 is applied topically to a treatment surface of the body in need of such treatment, and wherein optionally an additional skin care agent is also applied.

15. The composition of claims 1-6 for use as in claim 14, whereby the composition of claim (1) and the additional skin care agent are applied at substantially the same time.

## Patentansprüche

1. Zusammensetzung, umfassend (a) ein acyliertes Peptid der Formel (I)
R-X¹-X²-X³-X⁴ (I)
wobei R einen Fettsäurerest mit einer Kettenlänge darstellt, die geeignet ist, um zu unterstützen, dass die Verbindung der Formel (I) in Haut eindringt, X¹ eine basische Aminosäure oder eine hydrophobe aromatische Aminosäure darstellt, X² eine basische Aminosäure darstellt, X³ eine saure Aminosäure darstellt und X⁴ eine hydrophobe aliphatische Aminosäure oder polare neutrale Aminosäure, ein dermatologisch annehmbares Salz, Solvat und/oder Enantiomer davon darstellt; und (b) einen dermatologisch annehmbaren Träger.

2. Zusammensetzung nach Anspruch 1, wobei X¹ Phenylalanin, Tyrosin, Tryptophan, Histidin, Lysin oder Arginin darstellt; X² Histidin, Lysin oder Arginin darstellt; X³ Asparaginsäure oder Glutaminsäure darstellt; und/oder X⁴ Alanin, Valin, Methionin, Leucin, Isoleucin, Threonin, Glutamin, Cystein, Asparagin oder Serin darstellt.

3. Zusammensetzung nach Anspruch 1 oder Anspruch 2, wobei X¹ eine hydrophobe aromatische Seitenkette aufweist, X⁴ eine hydrophobe aliphatische Seitenkette aufweist oder wobei X¹ eine basische Aminosäure ist, X⁴ eine polare neutrale Seitenkette aufweist.

4. Zusammensetzung nach einem der vorhergehenden Ansprüche, wobei R einen Fettsäurerest der Formel R²C(O)- darstellt, wobei R² eine gesättigte oder ungesättigte geradkettige oder verzweigte Alkylgruppe mit 12 bis 24 Kohlenstoffatomen darstellt; wobei bevorzugt R Palmitoyl oder Myristoyl darstellt.

5. Zusammensetzung nach einem der vorhergehenden Ansprüche, wobei das acylierte Peptid der Formel (I) ein acyliertes Peptid der Formel (IA) oder (IB) sein kann
KRES (IA)
FREL (IB)
wobei die optionale Acylsubstituentengruppe ein Fettsäurerest mit einer Kettenlänge ist, die geeignet ist, um zu unterstützen, dass das Peptid der Formel (IA) oder (IB) in die Epidermis und Dermis der Haut eindringt.

6. Zusammensetzung nach einem der vorhergehenden Ansprüche, wobei das Peptid der Formel (I) der folgenden Formel sein kann
Palmitoyl-KRES (SEQ ID NO: 3) (ID)
Myristoyl-KRES (SEQ ID NO:5) (IID)
Palmitoyl-FREL (SEQ ID NO: 4) (IF)
Myristoyl-FREL (SEQ ID NO: 6) (IG).

7. Zusammensetzung nach einem der vorhergehenden Ansprüche, die ein zusätzliches Hautpflegemittel beinhaltet; wobei bevorzugt das zusätzliche Hautpflegemittel eines oder mehrere von einem Abschuppungsmittel, Mittel gegen Akne, Faltenreparaturmittel, Antioxidans, Radikalfänger, Chelatbildner, entzündungshemmenden Mittel, topischen Anästhetikum, Mittel gegen Cellulite, Flavonoid, antimikrobiellen Mittel, antifungalen Mittel, Sonnenschutzmittel und/oder Konditionierungsmittel ist, wobei bevorzugt das zusätzliche Hautpflegemittel ein Sonnenschutzmittel umfasst.

8. Zusammensetzung nach einem der vorhergehenden Ansprüche, die eine Hautpflegezusammensetzung ist, die (a) eine sichere und wirksame Menge eines Peptids der Formel (I) und (b) einen oder mehrere dermatologisch annehmbare Träger umfasst.

9. Zusammensetzung nach einem der vorhergehenden Ansprüche, die ein Peptid der Formel (I) in einer Konzentration von etwa 0,1 ppm bis etwa 1000 ppm umfasst.

10. Acyliertes Peptid der Formel (II)
R¹-X¹-X²-X³-X⁴ (II)
wobei R¹ einen Fettsäurerest mit einer Kettenlänge darstellt, die geeignet ist, um zu unterstützen, dass die Verbindung der Formel (II) in Haut eindringt, und wobei X¹, X², X³ und X⁴ wie in einem der Ansprüche 1 bis 4 in Bezug auf das Peptid der Formel (I) sind, ein dermatologisch annehmbares Salz, Solvat und/oder Enantiomer davon, ein dermatologisch annehmbares Salz, Solvat und/oder Enantiomer davon, das ein acyliertes Peptid der Formel (IIA) oder (IIB) ist
KRES (IIA)
FREL (IIB)
wobei die Acylsubstituentengruppe ein Fettsäurerest mit einer Kettenlänge ist, die geeignet ist, um zu unterstützen, dass das acylierte Peptid der Formel (IIA) oder (IIB) in Haut eindringt.

11. Acyliertes Peptid nach Anspruch 10, das von der folgenden Formel ist
Palmitoyl-KRES (SEQ ID NO: 3) (IIC).

12. Acyliertes Peptid nach Anspruch 10, wobei R¹ einen Fettsäurerest der Formel R₂C(O)-darstellt, wobei R₂ eine gesättigte oder ungesättigte geradkettige oder verzweigte Alkylgruppe mit 12 bis 24 Kohlenstoffatomen darstellt.

13. Acyliertes Peptid der Formel (I) nach einem der Ansprüche 1 bis 6 zur Verwendung beim Hemmen oder Vorbeugen der Entwicklung eines Hautzustands.

14. Zusammensetzung nach Anspruch 1-6 zur Verwendung beim Hemmen oder Vorbeugen der Entwicklung eines Hautzustands, wobei die Zusammensetzung nach einem der Ansprüche 1 bis 6 auf einen Menschen oder ein Tier aufgetragen wird, der/das einen solchen Schutz benötigt, wobei optional eine sichere und wirksame Menge der Zusammensetzung nach einem der Ansprüche 1 bis 7 topisch auf eine Behandlungsoberfläche des Körpers aufgetragen wird, die eine solche Behandlung benötigt, und wobei optional auch ein zusätzliches Hautpflegemittel aufgetragen wird.

15. Zusammensetzung nach Anspruch 1-6 zur Verwendung wie in Anspruch 14, wobei die Zusammensetzung von Anspruch (I) und das zusätzliche Hautpflegemittel im Wesentlichen gleichzeitig aufgetragen werden.

## Revendications

1. Composition comprenant (a) un peptide acylé de formule (I)
R-X¹-X²-X³-X⁴ (I)
dans laquelle R représente un résidu d'acide gras comportant une longueur de chaîne permettant d'assister la pénétration du composé de formule (I) dans la peau, X¹ représente un acide aminé basique ou un acide aminé aromatique hydrophobe, X² représente un acide aminé basique, X³ représente un acide aminé acide, et X⁴ représente un acide aminé aliphatique hydrophobe ou un acide aminé polaire neutre, un sel de qualité dermatologique, un solvate et/ou un énantiomère de celui-ci, et (b) un excipient de qualité dermatologique.

2. Composition telle que définie selon la revendication 1, dans laquelle X¹ représente phénylalanine, tyrosine, tryptophane, histidine, lysine ou arginine ; X² représente histidine, lysine ou arginine; X³ représente acide aspartique ou acide glutamique ; et/ou X⁴ représente alanine, valine, méthionine, leucine, isoleucine, thréonine, glutamine, cystéine, asparagine, ou serine.

3. Composition telle que définie selon la revendication 1 ou la revendication 2, dans laquelle X¹ comporte une chaîne latérale aromatique hydrophobe, X⁴ comporte une chaîne latérale aliphatique hydrophobe, ou dans laquelle X¹ est un acide aminé basique, X⁴ comporte une chaîne latérale polaire neutre.

4. Composition telle que définie selon l'une quelconque des revendications précédentes, dans laquelle R représente un résidu d'acide gras de formule R²C(O)- où R² représente un groupe alkyle à chaîne droite ou ramifié saturé ou non saturé comportant de 12 à 24 atomes de carbone ; de préférence R représente palmitoyl ou myristoyl.

5. Composition telle que définie selon l'une quelconque des revendications précédentes, dans laquelle le peptide acylé de formule (I) peut être un peptide acylé de formule (IA) ou (IB)
KRES (IA)
FREL (IB)
dans laquelle le groupe substituant acyle optionnel est un résidu d'acide gras comportant une longueur de chaîne permettant d'assister la pénétration du peptide de formule (IA) ou (IB) dans l'épiderme et le derme de la peau.

6. Composition telle que définie selon l'une quelconque des revendications précédentes, dans laquelle le peptide de formule (I) peut être de formule
Palmitoyl-KRES (SEQ ID NO: 3) (ID)
Myristoyl-KRES (SEQ ID NO:5) (IID)
Palmitoyl-FREL (SEQ. ID NO: 4) (IF)
Myristoyl-FREL (SEQ. ID NO: 6) (IG).

7. Composition telle que définie selon l'une quelconque des revendications précédentes, qui comprend un agent de soin cutané supplémentaire ; l'agent de soin cutané supplémentaire étant de préférence un ou plusieurs de : un agent desquamant, un agent anti-acné, un agent réparateur de rides, un antioxydant, un neutralisateur de radicaux, un chélateur, un agent anti-inflammatoire, un anesthésique topique, un agent anticellulite, un flavonoïde, un agent antimicrobien, un agent antifongique, un agent de protection solaire, et/ou un agent de conditionnement, de préférence dans laquelle l'agent de soin cutané supplémentaire comprend un agent de protection solaire.

8. Composition telle que définie selon l'une quelconque des revendications précédentes, qui est une composition de soin cutané comprenant (a) une quantité sûre et efficace d'un peptide de formule (I), et (b) un ou plusieurs excipients de qualité dermatologique.

9. Composition telle que définie selon l'une quelconque des revendications précédentes, qui comprend un peptide de formule (I) à une concentration d'environ 0,1 ppm à environ 1000 ppm.

10. Peptide acylé de formule (II)
R¹-X¹-X²-X³-X⁴ (II)
dans lequel R¹ représente un résidu d'acide gras comportant une longueur de chaîne permettant d'assister la pénétration du composé de formule (II) dans la peau, et dans lequel X¹, X², X³ et X⁴ sont tels que définis selon l'une quelconque des revendications 1 à 4 par rapport au peptide de formule (I), un sel de qualité dermatologique, un solvate et/ou un énantiomère de celui-ci, un sel de qualité dermatologique, un solvate et/ou un énantiomère de celui-ci qui est un peptide acylé de formule (IIA) ou (IIB)
KRES (IIA)
FREL (IIB)
dans lequel le groupe substituant acyle est un résidu d'acide gras comportant une longueur de chaîne permettant d'assister la pénétration du peptide acylé de formule (IIA) ou (IIB) dans la peau.

11. Peptide acylé tel que défini selon la revendication 10, qui est de formule
Palmitoyl-KRES (SEQ ID NO: 3) (IIC).

12. Peptide acylé tel que défini selon la revendication 10, dans lequel R¹ représente un résidu d'acide gras de formule R₂C(O)-, où R₂ représente un groupe alkyle à chaîne droite ou ramifié saturé ou non saturé comportant de 12 à 24 atomes de carbone.

13. Peptide acylé de formule (I) tel que défini selon l'une quelconque des revendications 1 à 6, destiné à être utilisé dans l'inhibition ou la prévention du développement d'une affection cutanée.

14. Composition selon les revendications 1 à 6 destinée à être utilisée dans l'inhibition ou la prévention du développement d'une affection cutanée, la composition telle que définie selon l'une quelconque des revendications 1 à 6 étant appliquée à un humain ou un animal ayant besoin de cette protection, éventuellement une quantité sûre et efficace de la composition telle que définie selon l'une quelconque des revendications 1 à 7 étant appliquée topiquement à une surface de traitement du corps nécessitant ce traitement, et éventuellement un agent supplémentaire de soin de la peau étant aussi appliqué.

15. Composition selon les revendications 1 à 6 destinée à être utilisée comme selon la revendication 14, la composition de la revendication (I) et l'agent supplémentaire de soin de la peau étant appliqués substantiellement en même temps.
